# EUROPEAN PATENT APPLICATION

(11) **EP 4 245 360 A1**
(43) Date of publication of application: **20.09.2023**
(21) Application number: 23157673.7
(22) Date of filing: 21.02.2023
(51) Int. Cl.: A61N 5/06, A61L 2/10, A61L 9/20

(54) **ULTRAVIOLET LIGHT IRRADIATION DEVICE**

(30) Priority: 16.03.2022 JP 2022040994
(71) Applicant: Ushio Denki Kabushiki Kaisha, Tokyo 100-8150 (JP)
(72) Inventor: FUJISAWA, Shigeki, Tokyo, 100-8150 (JP); YAGYU, Hideaki, Tokyo, 100-8150 (JP); KUNO, Akihiro, Tokyo, 100-8150 (JP)
(74) Representative: Tomerius, Isabel

(57) **Abstract**

An ultraviolet light irradiation device includes an optical filter. The optical filter has a transmission spectrum of zero-degree light including a first transmission band and a second transmission band that transmit the zero-degree light and a first restriction band that restricts transmission of the zero-degree light. The first transmission band is present within the wavelength band of 200 nm or more and less than 240 nm. The second transmission band is present within a wavelength band of more than 300 nm and less than 400 nm. The first restriction band is present over an entire wavelength range of at least 240 nm or more and 300 nm or less, and the first restriction band has an upper limit provided within a range of more than 300 nm and 380 nm or less.

## Description

### BACKGROUND OF THE INVENTION

### Field of the Invention

The present invention relates to an ultraviolet light irradiation device.

### Description of the Related Art

Ultraviolet light (UVC band) that is present in a wavelength range of 200 nm to 280 nm is known to inactivate pathogens such as viruses and bacteria that are found in the environment and is utilized in germicidal lamps. However, the ultraviolet light is also known to penetrate into the skin of a living body and damages cells in the skin. In addition, in recent years, research on the influence of ultraviolet light on a person has progressed, and it has been confirmed that even in the UVC band, the ultraviolet light in a wavelength band with a wavelength shorter than 240 nm is more likely to be absorbed by the skin surface layer or corneal epithelium as the wavelength becomes shorter, and cells inside the skin are less likely to be affected, thereby safety is improved. Therefore, an ultraviolet light irradiation device that actively irradiates a human body or an environment in which a person is present with the ultraviolet light having the wavelength band shorter than 240 nm has been put into practical use (see Patent Document 1).

### Prior Art Document

### Patent Document

Patent Document 1: JP 6908172 B2

### SUMMARY OF THE INVENTION

Recently, because of the influence of spread of the new coronavirus infection, there is an increasing need to inactivate pathogens such as bacteria and viruses that are found in the environment with the ultraviolet light. Therefore, further utilization of a device that emits the ultraviolet light in the wavelength range of 200 nm to 240 nm is desired. On the other hand, because ultraviolet light in a wavelength range of 240 nm to 280 nm is relatively highly harmful to the human body, the emission of a large amount of ultraviolet light in the wavelength range of 240 nm to 280 nm to the outside is required to be appropriately suppressed.

An object of the present invention is to provide an ultraviolet light irradiation device having improved pathogen inactivation capability while ensuring high safety for people and animals.

An aspect of an ultraviolet light irradiation device according to the present invention includes: a light source that emits ultraviolet light belonging to a wavelength band of 200 nm or more and less than 240 nm; and an optical filter disposed to allow the ultraviolet light to enter and including a dielectric multilayer film, in which the optical filter has a transmission spectrum of zero-degree light including a first transmission band and a second transmission band that transmit the zero-degree light and a first restriction band that restricts transmission of the zero-degree light, the zero-degree light being the ultraviolet light incident on the optical filter with an incident angle of zero degrees, the first transmission band is present within the wavelength band of 200 nm or more and less than 240 nm, the second transmission band is present within a wavelength band of more than 300 nm and less than 400 nm, and the first restriction band is present over an entire wavelength range of at least 240 nm or more and 300 nm or less, and the first restriction band has an upper limit provided within a range of more than 300 nm and 380 nm or less.

Although the ultraviolet light belonging to the wavelength band of 200 nm or more and less than 240 nm has only a small influence on the human body, in the case where the ultraviolet light is emitted to the environment where the people is present, there is a case where an irradiation dose of the ultraviolet light is required to be suppressed. For example, an ultraviolet light irradiation dose per day (8 hours) for a person is specified to be a threshold limit value (TLV) or less by American Conference of Governmental Industrial Hygienists (ACGIH) or JIS Z 8812 (Measuring methods of eye-hazardous ultraviolet radiation).

However, there is a case where the TLV specified for each wavelength is reviewed with the progress of research on the influence of ultraviolet light on the human body. For example, in the current ACGIH, the TLV of light having a wavelength of 222 nm is 22 mJ/cm² per day (8 hours). In the future, as the safety of light having the wavelength of 222 nm to the human body becomes clearer, the TLV is expected to be relaxed.

By the TLV being relaxed, the irradiation dose of the ultraviolet light belonging to the wavelength band of 200 nm or more and less than 240 nm can be increased in order to improve the inactivation capability. On the other hand, the ultraviolet light of 240 nm to 280 nm which is relatively highly harmful to the human body also increases. Although details are described later, the above-described ultraviolet light irradiation device can more appropriately suppress the ultraviolet light belonging to the wavelength band of 240 nm or more and less than 280 nm even when the irradiation dose of the ultraviolet light belonging to the wavelength band of 200 nm or more and less than 240 nm is increased.

Hereinafter, the description of the ultraviolet light irradiation device and the effects thereof are described in detail while the definition of terms and the like used in the present specification is shown.

In the present specification, the ultraviolet light belonging to the wavelength band of 200 nm or more and less than 240 nm emitted from the light source indicates that at least a part of the emission spectrum emitted by the light source exhibits intensity in the wavelength band of 200 nm or more and less than 240 nm. It is not always necessary that the intensity in the entire wavelength range of 200 nm or more and less than 240 nm is exhibited.

Examples of the light source that emits the ultraviolet light belonging to the wavelength band of 200 nm or more and less than 240 nm include a KrCl excimer lamp, a KrBr excimer lamp, and a light-emitting diode (LED) in which at least a part of the emitted light contains light exhibiting intensity in the wavelength band of 200 nm or more and less than 240 nm.

Light exhibiting intensity in the wavelength band of 200 nm or more and less than 240 nm is light having an action of inactivating pathogens and being less harmful people and animals. Therefore, the ultraviolet light irradiation device using the light source that emits such light can be installed in a space where people frequently come and go or a space where people work for a long time. The wavelength range that is safe for people and animals is preferably 200 nm or more and 237 nm or less, more preferably 200 nm or more and 235 nm or less, and still more preferably 200 nm or more and 230 nm or less. In the present specification, the light belonging to the wavelength band of 200 nm or more and less than 240 nm may be referred to as "target light".

As used herein, the term "pathogen" includes germs such as bacteria and fungi (mold), and viruses. Herein, "inactivation" is a concept of killing pathogens or causing pathogens to lose their infectivity or toxicity.

As described above, the optical filter including the dielectric multilayer film is disposed at the position where the ultraviolet light emitted from the light source is incident. The characteristics of the optical filter can be evaluated by a transmission spectrum in which a wavelength to be transmitted is represented on the horizontal axis and a relative intensity of light to be transmitted is represented on the vertical axis. Although details are described later, the transmission spectrum of the optical filter varies depending on the incident angle on the optical filter. Therefore, in the present specification, the characteristics of the optical filter are evaluated by distinguishing the incident angle. In the present specification, light incident on the optical filter at an incident angle of θ degrees is expressed as "θ-degree light". θ is zero degrees (0 deg) or more and less than 90 degrees (90 deg).

In the optical filter, the transmission spectrum of the zero-degree light in which the ultraviolet light is incident on the optical filter at the incident angle of zero degrees includes the first transmission band and the second transmission band that transmit the zero-degree light, and the first restriction band that restricts the transmission of the zero-degree light.

In the present specification, the transmittance is obtained by measuring a spectral spectrum of a light beam incident on the optical filter at a predetermined incident angle and a spectral spectrum of the light beam emitted from the optical filter with a spectrophotometer and obtaining (light intensity emitted from optical filter/light intensity incident optical filter) × 100 (%).

In the present specification, specific numerical values of the transmittance are not particularly mentioned, and when simply referred to as the "first transmission band" and the "second transmission band", the transmittance of the optical filter in the "first transmission band" and the "second transmission band" is 15% or more.

However, the transmittance in the first transmission band is preferably 30% or more, more preferably 50% or more, still more preferably 60% or more, still more preferably 70% or more, and still more preferably 80% or more.

However, the transmittance in the second transmission band is preferably 20% or more, more preferably 25% or more, more preferably 30% or more, more preferably 35% or more, more preferably 40% or more, more preferably 45% or more, and still more preferably 50% or more.

The transmittance in the second transmission band is preferably higher than the maximum transmittance in the first restriction band described later by 10% or more, more preferably higher by 15% or more, and still more preferably higher by 20% or more.

In the present specification, unless a specific numerical value is specified for the transmittance of ultraviolet light transmitted through the optical filter, the transmittance of the first restriction band that restricts the transmission of the optical filter is less than 5%.

However, the transmittance in the first restriction band is preferably 4% or less, more preferably 3% or less, still more preferably 2% or less, and still more preferably 1% or less. In particular, when the ratio of the harmful light emitted from the light source is large, it is desirable to adopt a smaller numerical value for the transmittance of the first restriction band.

The first transmission band is described. The first transmission band being present within the wavelength band of 200 nm or more and less than 240 nm means that the first transmission band exhibiting a predetermined transmittance or more (transmittance of 15% or more, unless otherwise specified) in the transmission spectrum of the optical filter is included in at least a part of the wavelength band of 200 nm or more and less than 240 nm. The first transmission band does not need to be present over the entire wavelength range of 200 nm or more and less than 240 nm.

The first restriction band is described. The first restriction band being present at least over the entire wavelength range of 240 nm or more and 300 nm or less means that a transmittance less than a predetermined transmittance (unless a specific numerical value is specified, transmittance of less than 5%) is present in the entire wavelength band of 240 nm or more and 300 nm or less in the transmission spectrum of the optical filter.

Among the light in the wavelength band of 240 nm or more and 300 nm or less, light in a wavelength band of 240 nm or more and less than 280 nm is the light in the wavelength band that is likely to adversely affect people and animals (hereinafter, the light may be referred to as the "harmful light"). The harmful light is the light not desirably emitted but the light that is inevitably emitted due to the nature of the light source. Therefore, the optical filter is used to restrict the transmission of the harmful light.

One of the features of the optical filter is that the first restriction band (e.g., the wavelength band having the transmittance of less than 5%) exceeds the wavelength band of the harmful light (240 nm or more and less than 280 nm) and extends to the wavelength band of 280 nm or more and 300 nm or less. Although details are described later, by expanding the first restriction band in the transmission spectrum for the zero-degree light to the entire wavelength range of 240 nm or more and 300 nm or less, the harmful light incident on the optical filter at a wide angle can also be appropriately restricted. As a result, even when the TLV is relaxed and the irradiation dose of ultraviolet light is increased, it is possible to effectively restrict harmful light incident at a wide angle.

One of the features of the optical filter is that a transmission spectrum of the zero-degree light has the second transmission band. The second transmission band is described. The second transmission band being present within the wavelength band of more than 300 nm and less than 400 nm means that a portion exhibiting a predetermined transmittance or more (transmittance of 15% or more, unless otherwise specified) is present in the transmission spectrum of the optical filter in at least a part of the wavelength band of more than 300 nm and less than 400 nm. The second transmission band does not need to be present over the entire wavelength range of more than 300 nm and less than 400 nm.

The operation and effect of having the second transmission band is described. In order to increase the upper limit value of the wavelength band restricting the transmission of light, it is effective to increase the film thickness of the dielectric multilayer film constituting the optical filter. However, if the film thickness of the dielectric multilayer film is unnecessarily increased, the intended transmittance of the first transmission band is lowered, and the light to be targeted is also attenuated. In addition, the increase in film thickness increases the cost of forming the dielectric multilayer film.

When the second transmission band is within the wavelength band of more than 300 nm and less than 400 nm in the transmission spectrum of the zero-degree light, the film thickness of the dielectric multilayer film is relatively thin, which can prevent a decrease in transmittance of the first transmission band.

Because the light in the wavelength band of more than 300 nm and less than 400 nm is neither the target light specified in the present specification nor the harmful light, conventionally, the transmission characteristic of the optical filter for the light in the above wavelength band has not been focused. An optical filter having the second transmission band within the wavelength band of 300 nm or more and less than 400 nm in the transmission spectrum of the zero-degree light is based on a design idea that is not an extension of a conventional design idea.

In the transmission spectrum of the zero-degree light of the optical filter, an optical filter having a cut-off upper limit wavelength within a wavelength band of 310 nm or more and less than 380 nm may be used. In the present specification, the "cut-off upper limit wavelength" is determined from a transmittance curve of a transmission spectrum represented by a first axis indicating transmittance [%] and a second axis perpendicular to the first axis and representing wavelength [nm]. Specifically, in the wavelength band sandwiched between the first restriction band and the second transmission band, the above wavelength refers to a wavelength at an intersection between a tangent of the transmittance curve at a position (e.g., the transmittance of 15%) where the transmission spectrum reaches the second transmission band and a reference line parallel to the second axis and passing through a transmittance of 0%. Exceptionally, when the wavelength at the intersection is within the first restriction band, an upper limit wavelength of the first restriction band is set as the "cut-off upper limit wavelength".

Note that the cut-off upper limit wavelength may be set within a wavelength band of 310 nm or more and 370 nm or less, or may be set within a wavelength band of 310 nm or more and 360 nm or less.

In the transmission spectrum of the zero-degree light of the optical filter, the upper limit wavelength of the first restriction band is set within a range of more than 300 nm and 380 nm or less. Here, the upper limit wavelength of the first restriction band may be 301 nm or more, 303 nm or more, 305 nm or more, or 307 nm or more. The more the upper limit wavelength of the first restriction band is set to a longer wavelength side, the wider the angle of light beam component in the emission of the harmful light can be appropriately restricted.

In the transmission spectrum of the zero-degree light of the optical filter, the first restriction band may further be present over the entire wavelength range of more than 300 nm and less than 310 nm, and the upper limit of the first restriction band may be provided within a range of 310 nm or more and 360 nm or less. With this configuration, the first restriction band is present over the entire wavelength range between the lower limit wavelength of 240 nm or less and the upper limit wavelength formed in the range of 310 nm or more and 360 nm or less. Here, the upper limit wavelength of the first restriction band may be 313 nm or more, 315 nm or more, or 317 nm or more. The more the upper limit wavelength of the first restriction band is set to a longer wavelength side, the wider the angle of light beam component in the emission of the harmful light can be appropriately restricted. The cut-off upper limit wavelength may be provided within a range of 320 nm or more and less than 370 nm.

In the transmission spectrum of the zero-degree light of the optical filter, the first restriction band may further be present over the entire wavelength range of 310 nm or more and less than 320 nm. The cut-off upper limit wavelength may be provided within a range of 310 nm or more and less than 360 nm, or may be provided within a range of 320 nm or more and less than 350 nm.

The second transmission band may be present over the entire wavelength range of 380 nm or more and less than 400 nm. The second transmission band may further be present over the entire wavelength range of 370 nm or more and less than 400 nm. The second transmission band may further be present over the entire wavelength range of 360 nm or more and less than 400 nm. The second transmission band may further be present over the entire wavelength range of 350 nm or more and less than 400 nm.

In the transmission spectrum of the zero-degree light of the optical filter, a second restriction band that does not transmit the zero-degree light may further be present within a wavelength band of 200 nm or more and less than 210 nm. This allows the emission of ultraviolet light in the vicinity of 200 nm and generating ozone in the atmosphere to be more appropriately restricted and ozone generation in the environment to be suppressed with higher accuracy.

In the optical filter, the transmission spectrum of a 50-degree light being the ultraviolet light incident on the optical filter with the incident angle of 50 degrees includes a third restriction band that restricts transmission of the 50-degree light over the entire wavelength range of 240 nm or more and less than 280 nm. This indicates that, even when the TLV is relaxed and the irradiation dose of ultraviolet light is increased, it is possible to effectively restrict the harmful light incident at a wide angle of 50 degrees.

The dielectric multilayer film may include a laminate in which high refractive index layers and low refractive index layers are alternately laminated, and the laminate may have a film thickness of 1.0 µm or more and 3.0 µm or less. In order to restrict the harmful light in a wide wavelength range in the transmission spectrum of the optical filter, it is desired that the laminate has a film thickness of at least 1.0 µm or more as a whole. However, as the film thickness increases, light belonging to a target wavelength band of 200 nm or more and less than 240 nm is less likely to be transmitted. Therefore, the laminate is desired to have a film thickness of 3.0 µm or less as a whole. Furthermore, the laminate may have a film thickness of 1.0 µm or more and 2.0 µm or less.

In the laminate, HfO₂ layers and SiO₂ layers are alternately laminated, and the total film thickness of all the HfO₂ layers included in the laminate may be 0.5 µm or more and less than 2.0 µm. In the dielectric multilayer film, HfO₂ functions as a high refractive index layer, and SiO₂ functions as a low refractive index layer.

The ultraviolet light irradiation device having improved pathogen inactivation capability while ensuring high safety for humans and animals can be provided.

Providing the ultraviolet light irradiation device corresponds to Goal 3 "Ensure healthy lives and promote well-being for all at all ages" of the UN-led Sustainable Development Goals (SDGs), and greatly contributes to Target 3.3 "By 2030, end the epidemics of AIDS, tuberculosis, malaria and neglected tropical diseases and combat hepatitis, water-borne diseases and other communicable diseases".

### BRIEF DESCRIPTION OF THE DRAWINGS

Fig. 1 is a diagram showing an ultraviolet light irradiation device according to an embodiment;
Fig. 2 is a diagram of the ultraviolet light irradiation device in Fig. 1 as viewed from the +Z side;
Fig. 3 is a cross-sectional view of the ultraviolet light irradiation device in Fig. 1 as viewed in the X direction;
Fig. 4 is a graph showing an emission spectrum of a light source of the present embodiment;
Fig. 5 is a diagram showing a relationship between irradiation time and an irradiation dose for target light and harmful light;
Fig. 6 is a diagram showing intensity distribution for each incident angle of light incident on an optical filter;
Fig. 7 is a diagram for explaining the incident angle;
Fig. 8 is a drawing explaining traveling of light emitted from a point light source in uniform light flux in all directions;
Fig. 9A is a transmission spectrum of the optical filter used in the present embodiment;
Fig. 9B is a transmission spectrum of an optical filter as a reference example; and
Fig. 10 is a diagram for explaining a method of obtaining a transmission spectrum from an optical filter.

### DETAILED DESCRIPTION OF THE PREFERRED EMBODIMENTS

The drawings are shown using an XYZ coordinate system as appropriate. The specification is described with reference to the XYZ coordinate system as appropriate. In describing directions in the present specification, in the case of distinguishing whether the direction is positive or negative, the positive or negative symbol is added, such as the "+X direction" or the "-X direction". In the case where there is no need to distinguish between positive and negative directions, the direction is simply described as the "X direction". Namely, in the present specification, in the case where the direction is simply described as the "X direction", both "+X direction" and "-X direction" are included. The same applies to the Y direction and the Z direction.

### [Outline of ultraviolet light irradiation device]

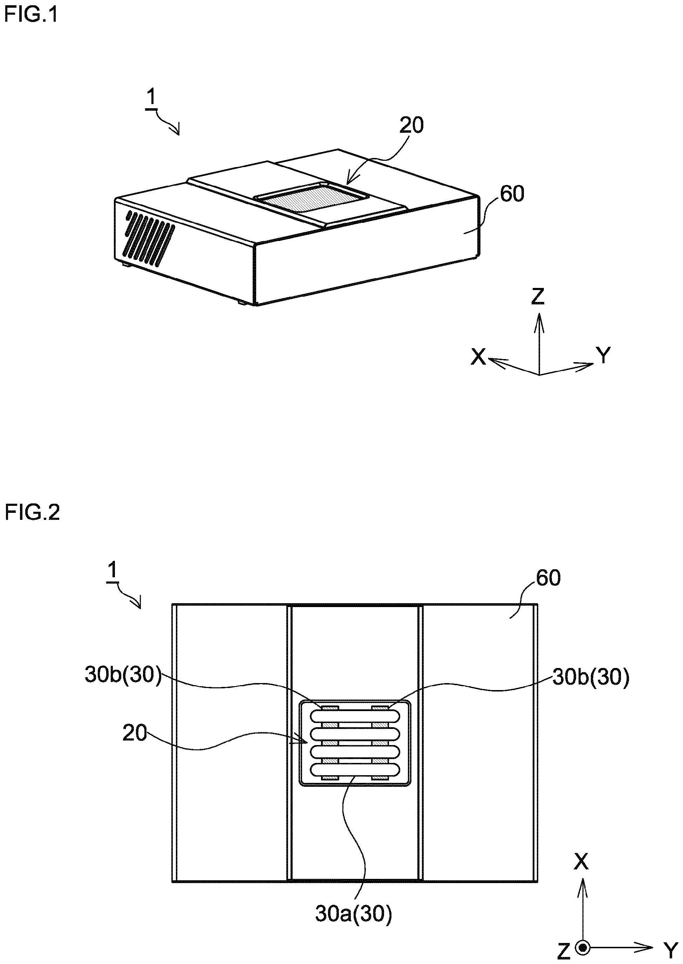

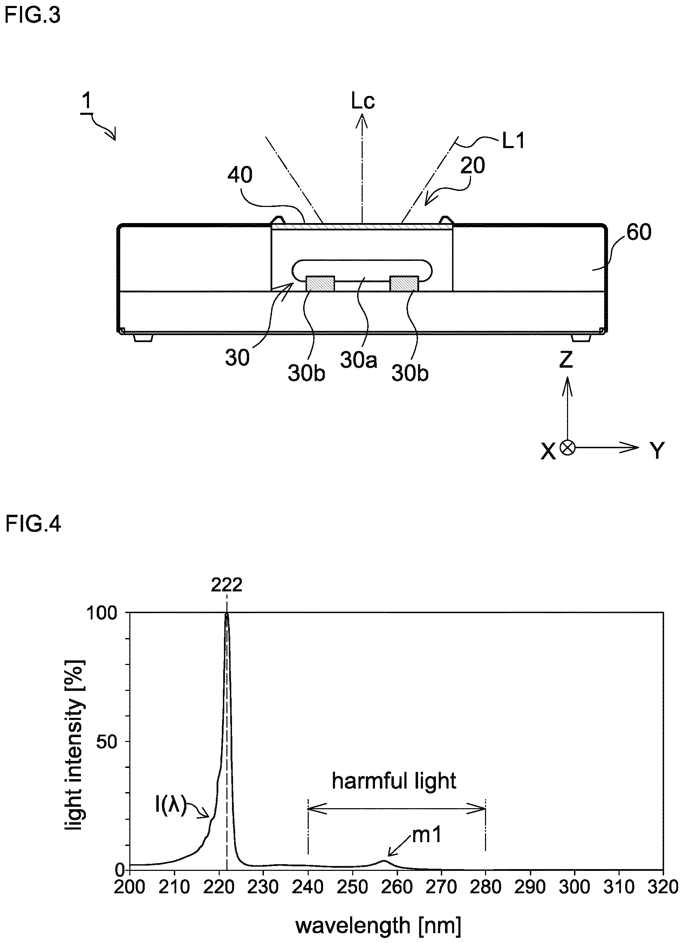

An outline of an embodiment of an ultraviolet light irradiation device is described with reference to Figs. 1 to 3. Fig. 1 is a schematic view showing an external appearance of an ultraviolet light irradiation device 1 according to the embodiment. Fig. 2 is a diagram of the ultraviolet light irradiation device 1 in Fig. 1 as viewed from the +Z side. Fig. 3 is a cross-sectional view of the ultraviolet light irradiation device 1 in Fig. 1 as viewed in the X direction.

The ultraviolet light irradiation device 1 of the present embodiment includes a casing 60, a light source 30 (see Figs. 2 and 3) accommodated in the casing 60, and a light extraction part 20 that extracts light emitted from the light source 30 to the outside of the casing 60.

As shown in Fig. 2, the light source 30 of the present embodiment is an excimer lamp that includes a plurality of light-emitting tubes 30a aligned in the X direction and a pair of electrodes 30b. Each light-emitting tube 30a extends in the Y direction. When a voltage is applied between the electrodes (30b, 30b), each light-emitting tube 30a emits light. The Z direction is a direction orthogonal to the X direction and the Y direction. The emitted light is extracted from the light extraction part 20.

In the ultraviolet light irradiation device 1, a length in a tube-axis direction (the Y direction) of the light-emitting tube 30a of the light source 30 is 70 mm, a distance between the light source 30 and an optical filter 40 is 8 mm, and a size of the optical filter 40 is (X, Y) = (60 mm, 45 mm). Note that each size configuration described herein is merely an example, and each size is optional.

As shown in Fig. 3, an optical axis Lc of ultraviolet light L1 emitted from the ultraviolet light irradiation device 1 is shown together with an arrow indicating an emission direction. In the present embodiment, the optical axis Lc extends along the Z axis. The optical filter 40 is disposed in the light extraction part 20. All the light emitted from the ultraviolet light irradiation device 1 is transmitted through the optical filter 40. Details of the optical filter 40 is described later.

The excimer lamp used in the present embodiment is a KrCl excimer lamp. The KrCl excimer lamp contains krypton (Kr) gas and chlorine (Cl) gas as light-emitting gas G1 in the light-emitting tube 30a. Fig. 4 is a graph showing an emission spectrum of the KrCl excimer lamp. The KrCl excimer lamp emits the ultraviolet light L1 in which the wavelength showing the maximum peak of light intensity I(λ) is 222 nm as shown in Fig. 4. In the emission spectrum of Fig. 4, the vertical axis represents the light intensity at each wavelength, the light intensity being represented using a standard value in which the light intensity at the wavelength of 222 nm is 100 (%).

As shown in Fig. 4, the light emitted from the KrCl excimer lamp shows a slight intensity even in the wavelength band (240 nm or more and less than 280 nm) of the harmful light that is likely to adversely affect people and animals. The emission spectrum in Fig. 4 has a small local maximum value m1 in the vicinity of 258 nm, but the light in the vicinity of m1 is light due to a phenomenon called chlorine emission in which excited Cl^{∗} collides with each other (* indicates an excited state) to emit light. In particular, when the Cl concentration sealed in the light-emitting tube 30a is high, a large amount of this light is emitted.

Nevertheless, as shown in Fig. 4, the light intensity of the harmful light is much smaller than the light intensity of the target light (e.g., 222 nm). Therefore, when the irradiation dose of ultraviolet light is small as a whole, the harmful light is not regarded as a problem as long as the optical filter that prevents transmission of harmful light is used. However, the optical filter transmits a small amount of harmful light. The present inventors have found that when the TLV is relaxed and the irradiation dose increases as a whole, a small amount of harmful light transmitted through the optical filter may be regarded as a problem depending on the operation condition of the ultraviolet light irradiation device 1.

### [Reason that harmful light is regarded as a problem]

The reason that a small amount of harmful light transmitted through the optical filter 40 is regarded as a problem is described. The present inventors have considered a case where the ultraviolet light irradiation device 1 increases the emission intensity or the actual irradiation time according to the relaxation of the TLV. However, as a result of the research, it has been found that the following problem occurs in the case of increasing the emission intensity or the actual irradiation time.

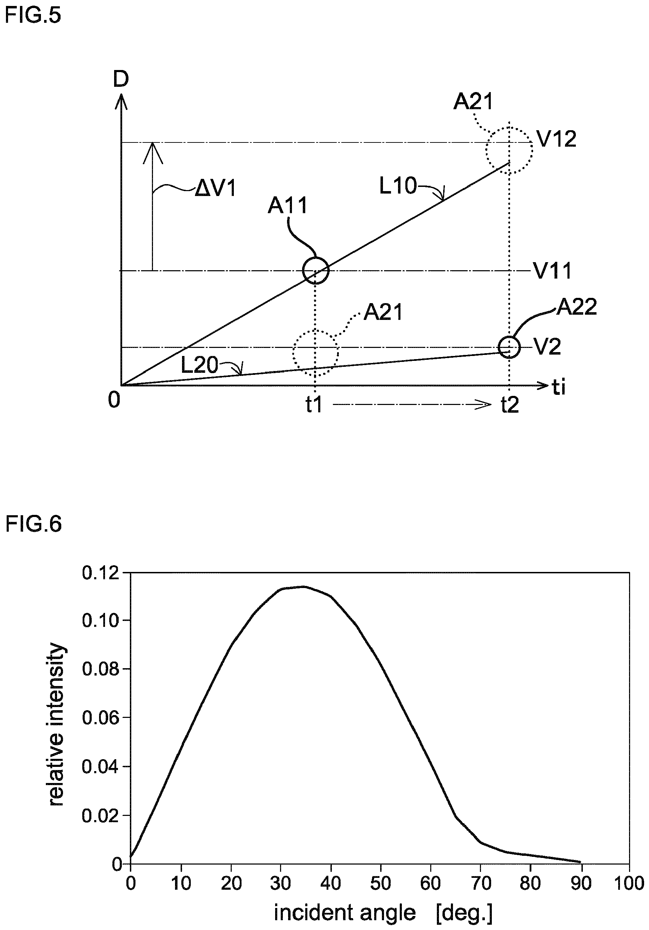

This problem is described with reference to Fig. 5. Fig. 5 is a graph showing the relationship between an irradiation time ti (horizontal axis) and a daily irradiation dose D (vertical axis) of target light L10 and harmful light L20. The irradiation time ti means the total sum of irradiation times in one day (integrated time). The ultraviolet light irradiation device 1 may perform not only the operation of always performing irradiation with the ultraviolet light in one day but also the operation of intermittently repeating irradiation and non-irradiation. The longer the irradiation time ti (unit: sec), the larger the irradiation dose D (unit: mJ/cm²).

It is assumed that, conventionally, the TLV of the target light L10 is defined to be V11 (mJ/cm²) and the TLV of the harmful light L20 is defined to be V2 (mJ/cm²). Conventionally, in order for the irradiation dose of the target light L10 not to exceed V11 (mJ/cm²), the time serving as an irradiation limit has been set to t1 (sec). That is, only a region A11 has been focused. Because the irradiation dose of the harmful light L20 does not reach V2 (mJ/cm²) at all, if only the region A11 is focused, a region A21 does not need to be focused.

Here, a scene is considered where the TLV of the target light is relaxed and the reference value of the irradiation dose D is set to be higher by ΔV1 from a conventional V11 (mJ/cm²) to a new V12 (mJ/cm²). As a result of considering extending the irradiation time to t2 (sec) according to the relaxation of the TLV, it has been found that the irradiation dose of the harmful light L20, which has not been necessary to be considered heretofore, approaches V2 (mJ/cm²) which is the TLV of the harmful light L20 (see a region A22).

Then, in order to set the irradiation time according to the relaxation of the TLV, it is necessary not only to prevent the irradiation dose of the target light L10 from exceeding V12 (mJ/cm²) (see the region A21) but also to prevent the irradiation dose of the harmful light L20 from exceeding V2 (mJ/cm²) which is the TLV of the harmful light (see the region A22). Therefore, when the TLV is relaxed and the irradiation dose increases, there is a possibility that the harmful light transmitted through the optical filter is regarded as a problem.

### [Optical filter taking light incident at wide angle into account]

Under the circumstances described above, the present inventors have studied the ultraviolet light irradiation device that can further suppress the harmful light transmitted through the optical filter. As a result of intensive studies, the present inventors have found that an optical filter that does not easily transmit light incident on the optical filter at a wide angle should be designed or selected. The reason for this is as follows.

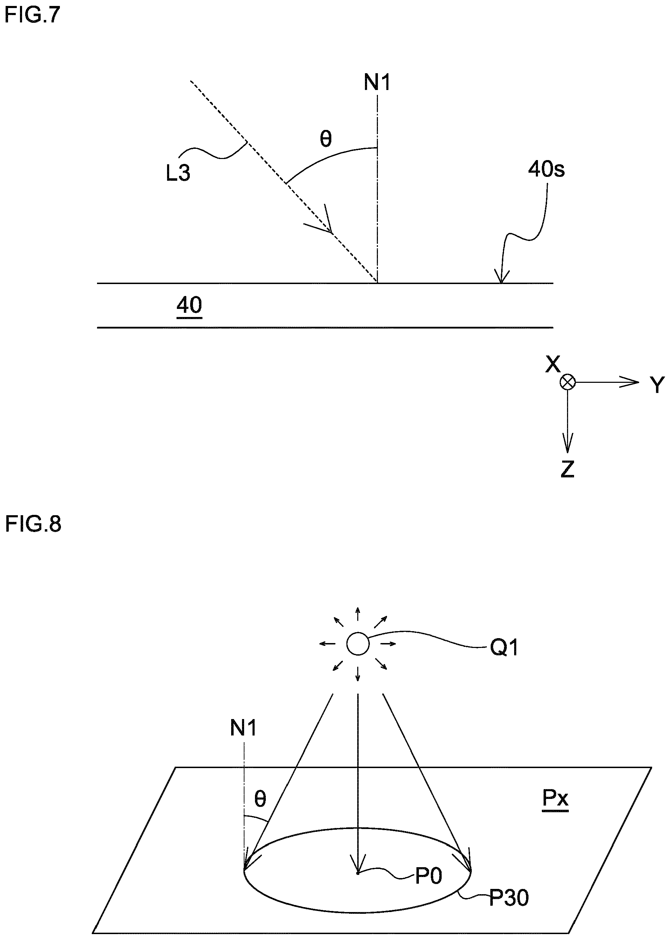

Fig. 6 shows a relative value (relative intensity) of the light intensity for each angular component of ultraviolet light with which the optical filter 40 is irradiated in the ultraviolet light irradiation device 1. This drawing is derived by calculation from the light distribution of ultraviolet light emitted from the ultraviolet light irradiation device 1. The horizontal axis represents the incident angle to the optical filter 40, and the vertical axis represents the relative intensity of ultraviolet light. As shown in Fig. 7, the incident angle is an angle θ formed between a normal line N1 of an incident surface 40s of the optical filter 40 and a light beam L3 incident on the optical filter 40.

From Fig. 6, it can be seen that most of the light beams exist in the range of the incident angle of 10 to 60 degrees, in particular, in the range of 20 to 50 degrees. In particular, it can be seen that the light beam component incident on the optical filter 40 at the incident angle of 30 to 40 degrees is much larger in amount than the light beam component incident on the optical filter 40 at the incident angle of zero degrees.

A reason of the light beam component incident on the optical filter 40 at the incident angle of 30 to 40 degrees is larger in amount than the light beam component incident on the optical filter 40 at the incident angle of zero degrees, is described with reference to Fig. 8. Fig. 8 is a drawing explaining traveling of light emitted from a point light source Q1 in uniform light flux in all directions. It is assumed that, as shown in Fig. 8, the light is emitted from the point light source Q1 in uniform light flux in all directions, and a plane Px is irradiated with a part of the light.

As shown in Fig. 8, in the plane Px, a region on which the light flux emitted from the point light source Q1 is incident at an incident angle θ of zero degrees is denoted as P0, and a region on which the light flux is incident at the incident angle θ of 30 degrees is denoted as P30. In the plane Px, as observed from Fig. 8, the region P30 is an annular region centered on the region P0, whereas the region P0 is only one point.

Moreover, as described above, in the case where the light is emitted from the point light source Q1 in the uniform light flux in all directions, it is observed that the total amount of light of the light flux incident on the entire region P30 forming the annular region are greater than that of the light flux incident only on one point of the region P0. In other words, assuming that the light source is a point light source, the total amount of light flux incident on a predetermined surface increases with an increase in incident angle θ from zero degrees. This means that the relative intensity of the light flux for each angle component is measured higher in the region P30 than in the region P0.

The light source 30 mounted on the ultraviolet light irradiation device 1 of the present embodiment can be regarded as an equivalent in which point light sources are aligned in the tube-axis direction of the light-emitting tubes 30a. Then, assuming the case where the aligned point light sources are individually considered, the light flux incident on the optical filter 40 is minimum when the incident angle θ is zero degrees, and with an increase in the incident angle θ from zero degrees, the total amount of light flux gradually increases.

The intensity of ultraviolet light incident on the optical filter 40 is proportional to the amount of light flux. The amount of light flux incident on the optical filter 40 increases with an increase in the incident angle θ from zero degrees. Once the incident angle θ reaches a magnitude that is large to a certain degree, the amount of light flux that cannot be incident on the optical filter 40 increases and thus the amount of light flux of ultraviolet light decreases. The incident angle θ at which the amount of incident light flux starts decreasing is adjusted by the distance between the light source 30 and the optical filter 40, the size of the light-emitting tube 30a of the light source 30, the area formed by the optical filter 40, and other factors.

The relative intensity for each angular component shown in Fig. 6 does not depend on the wavelength. For example, both the target light and the harmful light exhibit the same tendency. Based on the above findings, the present inventors have particularly studied an optical filter that can prevent transmission of a light beam incident at a wide range of incident angles.

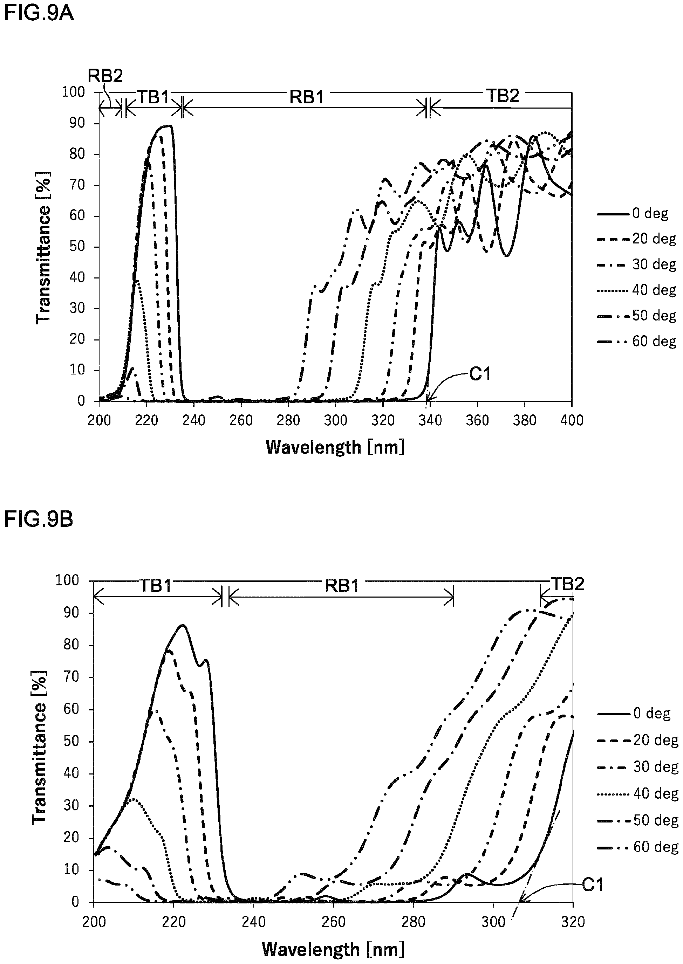

Features of the optical filter 40 is described with reference to Figs. 9A and 9B. Fig. 9A is a transmission spectrum of the optical filter 40 which is the present embodiment. Fig. 9B shows a transmission spectrum of an optical filter 90 as a reference example. Figs. 9A and 9B are both optical filters each including a dielectric multilayer film. Details of the dielectric multilayer film is described later.

Transmission spectra shown in Figs. 9A and 9B are described as follows. "0 deg" is a transmission spectrum of the zero-degree light incident on the optical filter (40, 90) at an incident angle of zero degree. Similarly, in the following, "20 deg" is a transmission spectrum of 20-degree light, "30 deg" is a transmission spectrum of 30-degree light, "40 deg" is a transmission spectrum of 40-degree light, "50 deg" is a transmission spectrum of 50-degree light, and "60 deg" is a transmission spectrum of 60-degree light.

As shown in Figs. 9A and 9B, the transmission spectrum tends to shift to the short wavelength side as the incident angle increases. This tendency is sometimes referred to as a "blue shift", following the fact that a blue wavelength band increases when a wavelength band of visible light shifts to the short wavelength side. The blue shift is considered to be caused by, as the incident angle of light incident on the dielectric multilayer film increases, an optical path length difference generated by a shift between a position of light reflected by the multilayer film and an incident position of light reciprocating in the film of the multilayer film.

First, the transmission spectrum of the optical filter 90 in Fig. 9B as a reference example is described. Based on the transmission spectrum of the zero-degree light, there are provided a first transmission band TB1 (here, the transmittance is 15% or more) that transmits the target light, a first restriction band RB1 (here, the transmittance is less than 5%) that restricts transmission of the harmful light, and a second transmission band TB2 (here, the transmittance is 15% or more). Because the first transmission band is present within the wavelength band of 200 nm or more and less than 240 nm, it can be seen that the target light is transmitted. Because the first restriction band RB1 is present over the entire wavelength range of 240 nm or more and less than 280 nm which is the wavelength band of the harmful light, transmission of the zero-degree light can be sufficiently prevented.

However, the transmission spectra of the 30-degree light, the 40-degree light, the 50-degree light, and the 60-degree light have a transmittance of 5% or more in 240 nm or more and less than 280 nm which is the wavelength band of the harmful light. That is, the optical filter 90 easily transmits the harmful light incident on the optical filter 90 at the wide angle (30 to 60 degrees). As shown in Fig. 6, a large amount of light enters the optical filter 90 at a wide angle, particularly 30 to 40 degrees. Therefore, even when the TLV is relaxed and the irradiation dose is to be increased, in the case of using the optical filter 90, a large amount of harmful light incident at a wide angle is transmitted through the optical filter 90, and thus the increase in the irradiation dose is restricted by the TLV of the harmful light.

Then, referring to Fig. 9B, because the zero-degree light of the first restriction band of 280 nm or more and less than 300 nm is not suppressed to the transmittance of less than 5%, it can be seen that the first restriction band RB1 does not satisfy the configuration of "being present over the entire wavelength range of 240 nm or more and less than 300 nm". Considering the fact that the transmission spectrum tends to shift to the short wavelength side as the incident angle increases, in the transmission spectrum of the zero-degree light, the harmful light incident on the optical filter 90 at a large angle cannot be prevented unless the first restriction band is extended not only to 240 nm or more and less than 280 nm, which is the wavelength band of the harmful light, but also to the longer wavelength side.

Next, the transmission spectrum of the optical filter 40 of the present embodiment in Fig. 9A is described. The transmission spectrum includes a first transmission band TB1 (here, the transmittance is 15% or more) that transmits the target light, a first restriction band RB1 (here, the transmittance is less than 5%) that restricts transmission of the harmful light, and a second transmission band TB2 (here, the transmittance is 15% or more). Because the first transmission band is present within the wavelength band of 200 nm or more and less than 240 nm, it can be seen that the target light is transmitted. Because the first restriction band RB1 is present over the entire wavelength range of 240 nm or more and less than 280 nm which is the wavelength band of the harmful light, transmission of the zero-degree light can be sufficiently prevented.

Unlike Fig. 9B, the optical filter 40 of Fig. 9A can restrict transmission of light of 240 nm or more and less than 280 nm, which is the wavelength band of the harmful light, even in the transmission spectra of the 30-degree light, the 40-degree light, the 50-degree light, and the 60-degree light. Because the optical filter 40 in Fig. 9A does not easily transmit the harmful light incident at the wide angle (30 to 60 degrees), the harmful light incident at the wide angle can be effectively restricted even when the TLV is relaxed and the irradiation dose of ultraviolet light is increased. Therefore, the irradiation dose does not need to be set according to the TLV of the harmful light, and the irradiation dose can be set in consideration of only the TLV of the target light.

Referring to Fig. 9A, in the transmission spectrum of the zero-degree light of the optical filter 40 that does not transmit the harmful light incident at the wide angle, the first restriction band RB1 is present over the entire wavelength range of 240 nm or more and less than 320 nm. Considering the fact that the transmission spectrum tends to shift to the short wavelength side as the incident angle increases, in the transmission spectrum of the zero-degree light, it can be said that, because the first restriction band is extended toward the longer wavelength side than the wavelength band of the harmful light, the transmission of light incident at the wide angle can be suppressed.

In the transmission spectrum of the zero-degree light, in the case of only extending the first restriction band to the longer wavelength side than the wavelength band of the harmful light, for example, it is sufficient to only increase the film thickness of the dielectric multilayer film. However, as described above, in order to maintain the transmittance of the first transmission band for transmitting the target light and to suppress the formation cost of the dielectric multilayer film, there is an upper limit value for the film thickness of the dielectric multilayer film.

In the above optical filter in which the film thickness of the dielectric layer film is made not too thick, the second transmission wavelength band appears on the longer wavelength side of the first restriction band in the transmission spectrum of the zero-degree light. That is, in the transmission spectrum of the zero-degree light, the optical filter 40 having the second transmission band TB2, which is present in the wavelength band of 320 nm or more on the longer wavelength side of the first restriction band, maintains the transmittance of the first transmission band for transmitting the target light and can suppress the formation cost of the dielectric multilayer film.

According to the intensive research of the present inventors, it has been found that, in the case of not disposing the optical filter, assuming the target light (light having a wavelength of 222 nm, hereinafter, the same applies) has the light intensity of 1, the light intensity of the target light in the optical filter in which the first restriction band is (not extended and) kept in the wavelength band of the harmful light (240 nm or more and less than 280 nm) is approximately in the range of 0.80 to 0.90, although it depends on the quality of the film formation of the dielectric multilayer film.

The optical filter in which the first restriction band is extended to 240 nm or more and less than 320 nm requires only a slight increase in the total film thickness as compared with the optical filter in which the first restriction band is not extended. Therefore, the light intensity of the target light in the optical filter having the restriction band extended is about the same as the light intensity of the target light in the optical filter having the restriction band not extended, or only decreases by a small amount if the light intensity is to be slightly decreased. That is, even when the upper limit wavelength of the first restriction band of the optical filter is extended to less than 320 nm or even less than 330 nm, the light intensity of the target light is easily maintained at a high level. Therefore, the optical filter having the first restriction band extended to 240 nm or more and less than 320 nm is effective because the amount of decrease in the light intensity of the target light can be suppressed while the transmission of the incident light at a wide angle can be suppressed.

On the other hand, in the optical filter in which the first restriction band is extended to 240 nm or more and 400 nm or less, the light intensity of the target light tends to decrease easily, and for example, decreases to 0.6 to 0.75 (the light intensity of the target light is set to 1). That is, due to the first restriction band is extended to 240 nm or more and less than 400 nm, the amount of the light intensity of the target light decreases by 0.15 to 0.20 (that is, 15 to 20%). Because the amount of decrease in the light intensity of the target light is too large, it is difficult to meet the purpose of improving the irradiation dose. Therefore, it is effective that the second transmission band is present within the wavelength band of more than 300 nm and less than 400 nm. In particular, by having the second transmission band present from the shorter wavelength side so as not to excessively extend the first restriction band in the wavelength band of more than 300 nm and less than 400 nm, it is possible to further suppress the amount of decrease in the light intensity of the target light.

Focusing now on a cut-off upper limit wavelength C1 appearing in the transmission spectrum of the zero-degree light, the cut-off upper limit wavelength C1 is present within a wavelength band of 320 nm or more and less than 350 nm in Fig. 9A while the cut-off upper limit wavelength C1 is present in a wavelength band less than 310 nm in Fig. 9B. The optical filter 40 in Fig. 9A can be distinguished from the optical filter 90 in Fig. 9B even when the cut-off upper limit wavelength C1 is used.

According to intensive research by the present inventors, it has been found that the amount of shift of the transmission spectrum to the short wavelength side as the incident angle increases is approximately 0.6 to 0.8 (nm/deg). That is, when the incident angle increases by one degree, the transmission spectrum at a specific incident angle shifts to the short wavelength side by 0.6 to 0.8 nm.

In consideration of the finding that the light beam component incident on the optical filter 40 at the incident angle of 30 to 40 degrees is the largest, which is found from Fig. 6, the optical filter 40 is desirably an optical filter considering light having an incident angle of 40 degrees or less. In the optical filter considering light having the incident angle of 40 degrees or less, the first restriction band in the transmission spectrum of the zero-degree light is present over the entire wavelength range of 240 nm or more and 300 nm or less, and in the transmission spectrum of the zero-degree light, the upper limit of the first restriction band is provided within a range of more than 300 nm and 380 nm or less. Further, the second transmission band is present within the wavelength band of more than 300 nm and less than 400 nm on the longer wavelength side of the first restriction band.

An optical filter may be used, which has a transmission spectrum in which the first restriction band is further present over the entire wavelength range of more than 300 nm and less than 310 nm, and the upper limit of the first restriction band is provided within a range of 310 nm or more and 360 nm or less. Still further, an optical filter may be used, which has the transmission spectrum in which the first restriction band is further present over the entire wavelength range of 310 nm or more and less than 320 nm.

In order to suppress transmission of light incident at still wider angle, not only considering light having an incident angle of 40 degrees or less, but also the use of an optical filter considering light having an incident angle of 50 degrees or less may be considered, and the use of an optical filter considering light having an incident angle of 60 degrees or less may be considered. In the optical filter considering light having an incident angle of 60 degrees or less, the first restriction band in the transmission spectrum of the zero-degree light is present over the entire wavelength range of 240 nm or more and less than 330 nm. For example, an optical filter in which the upper limit wavelength of the first restriction band is within a range of 330 nm or more and 360 nm or less can be used. Further, the second transmission band is present within the wavelength band of the upper limit wavelength of the first restriction band or more and less than 400 nm.

Furthermore, in the optical filter 40 having the transmission spectrum in Fig. 9A used in the present embodiment, transmission of ultraviolet light belonging to the wavelength band of 200 nm or more and 210 nm or less is restricted as compared with the optical filter 90 in Fig. 9B which is a reference example. For example, in the zero-degree light, there is a wavelength band in which the transmittance of ultraviolet light transmitted through the optical filter 40 is less than 5% present in the wavelength band of 200 nm or more and 210 nm or less. This wavelength band is referred to as a second restriction band RB2. By using the second restriction band RB2, the ultraviolet light in the vicinity of 200 nm and generating ozone in the atmosphere can be more appropriately restricted and the generation of ozone in the environment can be suppressed with higher accuracy. This also contributes to preventing ozone deterioration of peripheral members.

The above-described effect of the second restriction band RB2 is obtained by restricting ultraviolet light in the vicinity of 200 nm. In the second restriction band RB2, for example, the entire wavelength band of a wavelength of 200 nm or more and 202 nm or less may be restricted, the entire wavelength band of a wavelength of 200 nm or more and 205 nm or less may be restricted, the entire wavelength band of a wavelength of 200 nm or more and 207 nm or less may be restricted, and the entire wavelength band of a wavelength of 200 nm or more and 210 nm or less may be restricted.

The transmittance in the second restriction band RB2 is restricted to 5% or less. However, the transmittance in the second restriction band RB2 is preferably 4% or less, more preferably 3% or less, still more preferably 2% or less, and still more preferably 1% or less.

In addition, the optical filter applied to the present invention preferably has a narrow wavelength band sandwiched between the first restriction band RB1 and the second transmission band TB2 in the transmission spectrum of the zero-degree light. In other words, it is preferable to realize a cut-off wavelength obtained from an intersection point with a tangent having a larger inclination with respect to the transmittance curve. As a result, a border between the first restriction band RB1 and the second transmission band TB2 becomes clear, and the bandwidth of the first restriction band RB1 and the bandwidth of the transmission band can be specified more clearly. Specifically, the wavelength width between the first restriction band having a transmittance of less than 5% and the second transmission band having a transmittance of 15% or more is desirably 10 nm or less, and further desirably 5 nm or less. By narrowing the wavelength band sandwiched between the restriction band and the transmission band not only between the first restriction band RB1 and the second transmission band TB2, the restriction band and the transmission band are provided with high performance in a desired wavelength range, and thus, the characteristics becomes more suitable.

### [Method of obtaining transmission spectrum]

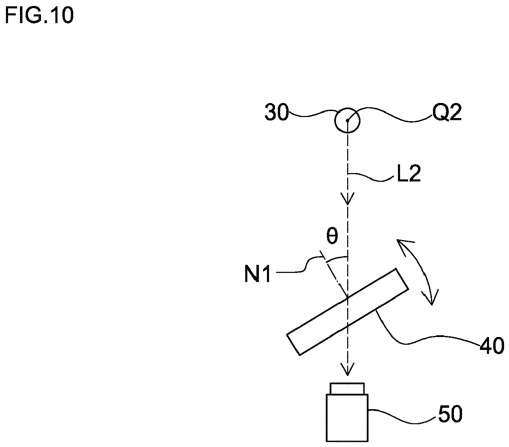

Fig. 10 is a diagram for explaining an example of a method of obtaining a transmission spectrum from the optical filter 40. The optical filter 40 is removed from the ultraviolet light irradiation device 1 and attached to an experimental system including a light source 30 and a spectrophotometer 50. As shown in Fig. 10, the optical filter 40 is disposed in an inclined manner to allow a light beam emitted from a center Q2 of the light source 30 toward the incident surface of the optical filter 40 to form a predetermined incident angle θ with respect to a normal line N1 of the optical filter 40. Directional light L2 is made incident on the optical filter 40 from the light source 30. The light intensity of the light transmitted through the optical filter 40 is measured by the spectrophotometer 50. By dividing the measured light intensity by the light intensity of the emitted light in a case where there is no optical filter, a transmission spectrum of the optical filter 40 at a predetermined incident angle θ is obtained. In addition, by measuring while changing the incident angle θ, a transmission spectrum for each incident angle can be obtained.

### [Structure of optical filter]

The optical filter 40 includes a dielectric multilayer film formed on a base material. The dielectric multilayer film includes a laminate in which high refractive index layers and low refractive index layers are alternately laminated. In the present embodiment, a laminate in which HfO₂ layers and SiO₂ layers are alternately laminated is used for the dielectric multilayer film of the optical filter 40. The laminate may be, for example, a laminate in which SiO₂ layers and Al₂O₃ layers are alternately laminated. The dielectric multilayer film in which the HfO₂ layers and the SiO₂ layers are alternately laminated can reduce the number of layers for obtaining the same wavelength-selective characteristics as compared with the dielectric multilayer film in which the SiO₂ layers and the Al₂O₃ layers are alternately laminated, and thus can increase the transmittance of the selected ultraviolet light. TiO₂, ZrO₂, or the like can also be used as the dielectric multilayer film.

The base material forming the dielectric multilayer film is made of a material that can transmit the target light. As the specific material for the base material, the following can be adopted which is a ceramic-based material such as silica glass, borosilicate glass, sapphire, magnesium fluoride material, calcium fluoride material, lithium fluoride material, and barium fluoride material, or a resin-based material such as a silicon resin and a fluororesin.

As described above, when the film thickness of the dielectric multilayer film is too thick, the transmittance of the first transmission band decreases, and the target light does not easily transmit through the dielectric multilayer film. When the film thickness is too thin, the harmful light (in particular, light beams incident on the optical filter at a wide angle) is transmitted. In consideration of such circumstances, the laminate of the dielectric multilayer film may have a film thickness of 1.0 µm or more and 3.0 µm or less as a whole, and may further have a film thickness of 1.0 µm or more and 2.0 µm or less. When the film thickness of the laminate is in this range, the harmful light (particularly, light beams incident on the optical filter at a wide angle) is easily suppressed while the transmittance of the target light is maintained in the first transmission band for transmitting the target light.

The transmission spectrum characteristics of the optical filter change not only by the film thickness of the entire laminate of the dielectric multilayer film but also by the combination of the materials of the dielectric multilayer film, the total film thickness for each material, the number of laminated layers, and the surface roughness of each film constituting the dielectric multilayer film. Therefore, for example, among the laminate in which the HfO₂ layers and the SiO₂ layers are alternately laminated, the total film thickness of all the HfO₂ layers may be specified to be 0.5 µm or more and less than 2 µm. By having the total film thickness of the HfO₂ layers to be 0.5 µm or more, an effect of preventing the harmful light is sufficiently obtained. Meanwhile, by having the total film thickness of the HfO₂ layers to be 2 µm or more, there is a case where the transmittance of the target light is restricted. Therefore, the total film thickness of the HfO₂ layers is desirably less than 2 µm. As described above, the specified optical filter can suppress the harmful light (particularly, light beams incident on the optical filter at a wide angle) while maintaining the transmittance of the target light in the first transmission band for transmitting the target light.

The embodiment of the ultraviolet light irradiation device has been described above. However, the present invention is not limited to the above embodiment, and various changes or modifications may be made to the above embodiment without departing from the spirit of the present invention.

In the present embodiment, a KrCl excimer lamp is employed as the light source 30, but the present invention is not limited thereto. An excimer lamp filled with another gas (for example, an excimer lamp filled with Kr gas and Br gas and having a maximum intensity in the vicinity of 207 nm) may be adopted as the light source 30. In addition, a solid light source such as an LED may be adopted as the light source 30. Even when the excimer lamp having another gas filled therein or the solid light source such as an LED is adopted, the present invention is established.

For example, the light source 30 may be a light source that emits ultraviolet light having a main emission wavelength within a range of 200 nm or more and less than 240 nm. The light source 30 is not limited to an excimer lamp, and a solid light source such as an LED may be adopted as the light source 30. For example, an AlGaN-based LED or an MgZnO-based LED having a main emission wavelength of less than 240 nm can be adopted as the light source 30. Furthermore, in a case of using a coherent light source as the light source 30, a light source that emits coherent ultraviolet light from a gas laser or a solid laser element may be used, or a light source that uses a wavelength conversion element may be used in which light emitted from a gas laser or a solid laser element is used to newly generate coherent light having different wavelengths. As the wavelength conversion element, for example, a non-linear optical crystal that multiplies the frequency of light emitted from the laser element to generate a high-order harmonic waves such as second harmonic generation (SHG) waves or third harmonic generation (THG) waves can be used. Furthermore, the light source 30 may be a light source utilizing a fluorescent body that emits ultraviolet light having a main emission wavelength within a range of 200 nm or more and less than 240 nm. The "main emission wavelength" described here indicates, in a case where a wavelength range Z(λ) of ± 10 nm with respect to a certain wavelength λ is defined on the emission spectrum of the light source 30, a wavelength λi in the wavelength range Z(λi) showing an integrated intensity of 40% or more with respect to the total integrated intensity in an emission spectrum.

### DESCRIPTION OF REFERENCE SIGNS

- 1: Ultraviolet light irradiation device
- 20: Light extraction part
- 30: Light source
- 30a: Light-emitting tube
- 30b: Electrode
- 40: Optical filter
- 40s: Incident surface
- 50: Spectrophotometer
- 60: Casing
- 90: Optical filter

## Claims

1. An ultraviolet light irradiation device comprising:
a light source that emits ultraviolet light belonging to a wavelength band of 200 nm or more and less than 240 nm; and
an optical filter disposed to allow the ultraviolet light to enter and including a dielectric multilayer film, wherein
the optical filter has a transmission spectrum of zero-degree light including a first transmission band and a second transmission band that transmit the zero-degree light and a first restriction band that restricts transmission of the zero-degree light, the zero-degree light being the ultraviolet light incident on the optical filter with an incident angle of zero degrees,
the first transmission band is present within the wavelength band of 200 nm or more and less than 240 nm,
the second transmission band is present within a wavelength band of more than 300 nm and less than 400 nm, and
the first restriction band is present over an entire wavelength range of at least 240 nm or more and 300 nm or less, and the first restriction band has an upper limit provided within a range of more than 300 nm and 380 nm or less.

2. The ultraviolet light irradiation device according to claim 1, wherein the first restriction band is further present over an entire wavelength range of more than 300 nm and less than 310 nm, and the first restriction band has the upper limit provided within a range of 310 nm or more and 360 nm or less.

3. The ultraviolet light irradiation device according to claim 2, wherein the first restriction band is further present over an entire wavelength range of 310 nm or more and less than 320 nm.

4. The ultraviolet light irradiation device according to claim 1, wherein the second transmission band is further present over an entire wavelength range of 380 nm or more and less than 400 nm.

5. The ultraviolet light irradiation device according to claim 4, wherein the second transmission band is further present over an entire wavelength range of 360 nm or more and less than 380 nm.

6. The ultraviolet light irradiation device according to claim 5, wherein the second transmission band is further present over an entire wavelength range of 340 nm or more and less than 360 nm.

7. The ultraviolet light irradiation device according to any one of claims 1 to 6, wherein the transmission spectrum of the zero-degree light further has a second restriction band that restricts transmission of the zero-degree light in a wavelength band of 200 nm or more and 210 nm or less.

8. The ultraviolet light irradiation device according to any one of claims 1 to 7, wherein the optical filter has a transmission spectrum of a 50-degree light being the ultraviolet light incident on the optical filter with an incident angle of 50 degrees, the transmission spectrum having a third restriction band that restricts transmission of the 50-degree light over an entire wavelength range of 240 nm or more and less than 280 nm.

9. The ultraviolet light irradiation device according to any one of claims 1 to 8, wherein
the dielectric multilayer film includes a laminate in which high refractive index layers and low refractive index layers are alternately laminated, and
the laminate may have a film thickness of 1.0 µm or more and 3.0 µm or less.

10. The ultraviolet light irradiation device according to claim 9, wherein the laminate has a film thickness of 1.0 µm or more and 2.0 µm or less.

11. The ultraviolet light irradiation device according to claim 9 or 10, wherein
the laminate has HfO₂ layers and SiO₂ layers alternately laminated, and
the HfO₂ layers that are all included in the laminate have a total thickness of 0.5 µm or more and less than 2.0 µm.
